# EUROPÄISCHE PATENTSCHRIFT

(11) **EP 3 142 612 B1**
(45) Veröffentlichungstag und Bekanntmachung des Hinweises auf die Patenterteilung: **18.04.2018**
(21) Anmeldenummer: 15726518.2
(22) Anmeldetag: 08.05.2015
(51) Int. Cl.: A61F 2/66, A61F 2/78, A61F 2/50

(54) **GELENKLOSER PROTHESENFUSS**
JOINTLESS PROSTHETIC FOOT
PROTHÈSE DU PIED SANS ARTICULATION

(30) Priorität: 12.05.2014 DE 102014006744
(43) Veröffentlichungstag der Anmeldung: 22.03.2017
(73) Patentinhaber: Otto Bock HealthCare GmbH, 37115 Duderstadt (DE)
(72) Erfinder: BOITEN, Herman, 37085 Göttingen (DE); NÖRTHEMANN, Jens, 37115 Duderstadt (DE); CREMER, Andrea, 50126 Bergheim (DE)
(74) Vertreter: Gramm, Lins & Partner Patent- und Rechtsanwälte PartGmbB
(86) Internationale Anmeldenummer: PCT/DE2015/100187
(87) Internationale Veröffentlichungsnummer: WO 2015/172767

(56) Entgegenhaltungen:
- EP-A1- 0 793 949
- US-A1- 2009 082 878
- US-B1- 8 535 390
- US-B2- 7 520 904

## Beschreibung

Die Erfindung betrifft einen gelenklosen Prothesenfuß mit einer Blattfeder, die einen distalen Auftrittsabschnitt, einen Verbindungsabschnitt und einen proximalen, sich in anteriorer-posteriorer Richtung erstreckenden Anschlussabschnitt aufweist, an dem ein Anschlussadapter festlegbar ist. Ein solcher Prothesenfuß ist insbesondere als Sportfuß geeignet.

Sportfüße sind in der Regel als eine einteilige Blattfeder aus einem faserverstärkten Kunststoffmaterial ausgebildet. Die Anbindung an einen Schaft, die auch bei Unterschenkelprothesen möglich sein soll, führt bei einer Belastung dazu, dass die Spitze der Blattfeder sich in Bezug auf den Schaft nicht nur nach oben, also in Vertikalrichtung, sondern durch eine Biegung der Feder auch nach vorne bewegt. Beim Auftreten führt dies dazu, dass das Bein gegenüber dem Boden nach hinten gedrückt wird. Auch bei einer Freigabe der in der Feder gespeicherten Energie kann es zu einer posterior-gerichteten Komponente kommen, so dass die Richtung der Energiefreigabe nicht zu 100 % mit der Laufrichtung des Sportlers übereinstimmt.

Unter der Bezeichnung Flex-Run vertreibt die Firma Össur einen Sportfuß mit einer C-förmig gebogenen Feder, an deren oberen Ende ein Anschlussadapter festgelegt ist.

Die Chas A Blatchford & Sons Ltd. vertreibt unter der Bezeichnung "endolite Blade XT" einen Prothesenfuß für Breitensportler und Läufer mit einer Vorderfußfeder, die einen im Wesentlichen waagerechten Kopfabschnitt und eine nach außen konvex geformte, einteilige Feder aufweist, die im unteren Bereich durch einen Schlitz zweigeteilt ist. In dem vorderen Bereich der Feder ist ein Sohlenschutz und eine Fersenfeder über zwei Schrauben befestigt, über einen Keil kann die Federhärte der Fersenfeder eingestellt werden. Das Einsetzen des Fersenkeils hat zur Folge, dass die Fersenfeder weniger flexibel reagiert. Ein Proximaladapter ist in einem Aufnahmeschlitz verschieblich festlegbar geführt.

Die Firma Freedom Innovations vertreibt einen Sportfuß unter der Bezeichnung "Nitro Running Foot" mit einem vertikalen Aufnahmebereich, an dem ein Proximaladapter festgelegt ist. Von dem Proximaladapter erstreckt sich eine einteilige Blattfeder in einem Bogen bis zu einem Auftrittsbereich. Die spezielle Blattfeder ist leicht nach oben geneigt.

Die US 8,535,390 B1 betrifft eine abnehmbare Sohle für einen Sportprothesenfuß. Die Sohle wird an einem distalen Ende einer Blattfeder befestigt, an der ein festes Anschlussmittel zur Anbindung an den Nutzer der Prothese angeordnet ist.

Die EP 0 793 949 A1 betrifft einen gelenklosen Kunstfuß mit einem federelastischen Fußeinsatz mit einer sohlenseitigen Blattfeder, an dessen Unterseite im vorderen und hinteren Bereich Polster angeordnet sind. Ein rohrförmiges, zylindrisches Segment mit einer horizontalen Zylinderachse ist im Fersenbereich an der Blattfeder befestigt. In einem proximalen Abschnitt des zylindrischen Segmentes ist eine Aufnahme für ein Unterschenkelrohr in einem Schlitz gelagert.

Die US 2006/0030950 A1 betrifft einen Prothesenfußeinsatz mit einstellbaren Eigenschaften. Eine Basisfeder mit nach unten gekrümmten Fersen- und Vorderfußabschnitten sowie einem bogenförmigen Verbindungsabschnitt ist mit einem Längsschlitz versehen, in dem ein Verbindungsteil zu einem Unterschenkelschaft verschieblich gelagert werden kann.

Die US 5,653,768 A betrifft einen zweifach schwenkbar gelagerten Prothesenfuß mit einer ersten Blattfeder, die sich vom Fersenbereich zunächst nach vorne erstreckt, eine Krümmung durchläuft und anschließend durch eine Ausnehmung in einer zweiten, umgekehrt gekrümmten Blattfeder durch einen Wadenbereich erstreckt. Die zweite Blattfeder erstreckt sich vom Schienbeinbereich in einem Bogen durch den Knöchelbereich in den Vorderfußbereich.

Die FR 2 626 463 A1 betrifft einen gelenklosen Prothesenfuß mit einer Blattfeder, die in einer Prothesenfußkosmetik eingebettet ist. Ein kreisförmiger Fersenabschnitt erstreckt sich von einem Mittelfußbereich bis zu einem oberen Anschlussbereich und von dort schräg nach vorne in einen Vorderfußbereich. Im oberen Anschlussbereich ist ein Adapter zur Befestigung eines Unterschenkelrohrs in einem Langloch auf der Oberfläche der Blattfeder verschieblich gelagert.
Aufgabe der vorliegenden Erfindung ist es, einen Prothesenfuß bereitzustellen, der einfach an die unterschiedlichen Bedürfnisse eines Nutzers anpassbar ist und die Wirkungslinie näher an den Körperschwerpunkt heranzubringen, um eine möglichst effiziente Nutzung des Prothesenfußes zu ermöglichen.

Erfindungsgemäß wird diese Aufgabe durch einen gelenklosen Prothesenfuß mit den Merkmalen des Hauptanspruches gelöst. Vorteilhafte Ausgestaltungen und Weiterbildungen der Erfindung sind in den Unteransprüchen, der Beschreibung und den Figuren offenbart.

Der gelenklose Prothesenfuß mit einer Blattfeder, die einen distalen Auftrittsabschnitt, einen Verbindungsabschnitt, der sich schräg aufwärts nach hinten erstrecken kann, und einen proximalen, sich in anteriorer-posteriorer Richtung erstreckenden Anschlussabschnitt aufweist, an dem ein Anschlussadapter festlegbar ist, sieht vor, dass der Anschlussadapter entlang einer konvex geformten Bahn in anterior-posterior Richtung verstellbar gelagert ist. Durch die verstellbare, bevorzugt stufenlos einstellbare Lagerung des Anschlussadapters entlang einer Bahn, die konvex, also nach außen gewölbt gekrümmt ist, ist es möglich, dass bei einer Relativverlagerung des Anschlussadapters zu der Blattfeder in anteriorer-posteriorer Richtung die Position des Auftrittbereiches oder der Auftrittslinie zu der Lotrechten des Anschlussadapters im Wesentlichen konstant bleibt, also keine Verschiebung des Auftrittsbereiches in anterior-posterior-Richtung erfolgt. Dadurch ist es möglich, dass eine erhöhte Variabilität der Krafteinleitung aufgrund der Veränderung des Krafthebels zu bewirken, ohne den Aufbau der Prothese zu verändern. Insbesondere wird bei einer Verschiebung der Blattfeder in anteriorer Richtung keine Verlagerung des Auftrittsbereiches in anteriore Richtung zu dem Körperschwerpunkt bewirkt, was in einer Verschiebung der Wirkungslinie vor den Körperschwerpunkt resultieren würde, wodurch der Prothesennutzer ausgebremst werden würde. Darüber hinaus ist es neben der konstanten oder nahezu konstanten Position des Auftrittsbereiches und der Vermeidung einer Vergrößerung einer rückwärts gerichteten Kraftkomponente die Höhe des Prothesenfußes beizubehalten, wodurch sich für den Nutzer trotz Veränderung der Federeigenschaft keine spürbare Veränderung der Prothesenabmessung ergibt. Die Bahn, auf der der Anschlussadapter insbesondere verschieblich gelagert ist, weist zumindest bereichsweise einen Krümmungsradius auf, der in dem Auftrittsabschnitt bei der Verstellung der Blattfeder mündet. Fällt der Abrollpunkt oder die Abrolllinie im Auftrittsabschnitt mit dem Mittelpunkt der Krümmung zusammen, bildet der Radius die Form der Bahnkrümmung, entlang der der Anschlussadapter verschoben werden kann.

Bei einer direkten Lagerung des Anschlussadapters auf der Blattfeder kann der der Anschlussabschnitt unmittelbar konvex geformt sein, um die gewünschte Bahnkurve auszubilden, Die Blattfeder wird dann bei der Verstellung unter dem Adapter in anteriorer oder posteriorer Richtung, einfacher nach vorn oder hinten verschoben und der Adapter in der gewünschten Position fixiert. Wird der Adapter über eine Anschlussadapterlagerung an der Blattfeder befestigt, kann diese ebenfalls eine nicht geradlinige Verstellung zulassen, so dass eine Verstellung an der Anschlussadapterlagerung allein oder in Ergänzung mit einer ungeraden Ausgestaltung des Anschlussabschnittes zu einer im Wesentlichen konstanten Prothesenhöhe bei unterschiedlichen Positionen des Anschlussadapters an der Blattfeder führt. Sowohl der Anschlussabschnitt als auch die Anschlussadapterlagerung können konvex geformt sein, die Anschlussadapterlagerung kann eine gekrümmte Führungsbahn für den Anschlussadapter aufweisen, wobei die Krümmungen des Anschlussabschnittes und/oder der Anschlussadapterlagerung nicht konstant sein müssen. Die Krümmungen sind so ausgebildet oder ergänzen sich dergestalt, dass der Anschlussadapter selbst auf einer im Wesentlichen konstanten Höhe bei einer Verstellung verbleibt und der Auftrittsbereich bei einer Verstellung im Wesentlichen unverändert bleibt. Liegt der Drehpunkt der Blattfeder im Auftrittsabschnitt in der Lotrechten zu dem Anschlussadapter und weist die Auflagefläche des Auftrittsabschnittes eine konstante Krümmung auf, dann kann die Krümmung des Anschlussabschnittes und/oder die Krümmung der Anschlussadapterlagerung konstant sein. Liegt der Auftrittpunkt oder die Auftrittslinie vor oder hinter der Lotrechten und ist z.B. der Auftrittsabschnitt im Abrollbereich bei der Verstellung mit einem konstanten Krümmungsradius versehen, ist die Krümmung des Anschlussabschnittes und/oder der Anschlussadapterlagerung dergestalt, dass der Höhenunterschied ausgeglichen wird, die Prothesenfußhöhe konstant bleibt und der Auftrittspunkt oder die Auftrittslinie wie vorher eingestellt vor oder hinter der Lotrechten liegt. Der Aufbau verändert sich nicht.

In dem Anschlussabschnitt und/oder der Anschlussadapterlagerung kann ein sich in anteriorer-posteriorer Richtung erstreckender Aufnahmeschlitz für den Anschlussadapter angeordnet oder ausgebildet sei. Durch die Anordnung des Aufnahmeschlitzes in dem Anschlussabschnitt oder der Anschlussadapterlagerung ist es möglich, eine erhöhte Variabilität der Krafteinleitung aufgrund der Veränderung des Krafthebels bei gleichzeitiger stufenloser Verlagerbarkeit und guter Führungsgenauigkeit zu bewirken. Neben einem Aufnahmeschlitz kann der Anschlussadapter an einem Führungssteg oder direkt an der Blattfeder befestigt sein, beispielsweise über eine Klemmung.

Eine Weiterbildung der Erfindung sieht vor, dass der Krümmungsradius in einem Abrollbereich in dem Auftrittsabschnitt mündet.

Eine vorteilhafte Weiterbildung sieht vor, dass der Verbindungsabschnitt konkav geformt ist, so dass sich nach dem Auftrittsabschnitt die Blattfeder von dem Boden weg wölbt, was dazu führt, dass der Verbindungsbereich auch bei einer vergrößerten Schrittlänge nicht in Kontakt mit dem Boden treten kann. Der Bodenkontakt der Blattfeder erfolgt also nur im Auftrittsabschnitt, nicht jedoch in dem sich posterior und proximal daran anschließenden Verbindungsabschnitt. Dadurch ist es möglich, den Verbindungsabschnitt frei von jeglichem Bodenkontakt zu halten, so dass eine definierte Zuordnung der jeweiligen Funktionen in den jeweiligen Abschnitten hergestellt werden kann. Der Auftrittsabschnitt ist der einzige Bereich, der Bodenkontakt hat, der Anschlussabschnitt definiert die Zuordnung des Anschlussadapters zu der Blattfeder und der Verbindungsabschnitt stellt die federnde, sich posterior und proximal erstreckende Verbindung zwischen diesen beiden Abschnitten her. Durch die konvexe Form des Anschlussabschnittes ist es bei einer Rotation um den Auftrittspunkt bzw. der Auftrittslinie in dem konvex, nach außen in Richtung auf den Bogen gewölbt geformten Auftrittsabschnitt möglich, eine Vergrößerung oder Verringerung der Hebellänge der Blattfeder zu bewirken, ohne dass die Fußhöhe oder die Position des Auftrittsbereiches verändert wird. Wird eine größere Hebellänge gewünscht, beispielsweise um bei entspannten Joggen ein weicheres Einfedern zu ermöglichen, wird der Anschlussadapter nach vorne verschoben, wird der Anschlussadapter weiter nach hinten verschoben, versteift sich der Feder, was für den Sprint bevorzugt ist. Der bevorzugte Verlauf der Blattfeder ist somit von dem Anschlussadapter gesehen zunächst nach oben von dem Boden weg konvex geformt, anschließend in dem Verbindungsabschnitt von dem Boden weg konkav geformt und schließlich konvex zum Boden hin im Auftrittsabschnitt geformt, um dort einen definierten Drehpunkt bzw. eine definierte Drehachse im Auflagebereich des Auftrittsabschnittes bereitzustellen, die aufgrund der konvexen Ausgestaltung der Verschiebebahn des Anschlussadapters nahezu unverändert relativ zu der Lotrechten des Anschlussadapters bleibt. Durch den konkaven Verbindungsabschnitt wird sichergestellt, dass der Bodenkontakt immer im vorderen Auftrittsbereich stattfindet. Wenn bei einem stark angewinkelten Auftreten zuerst der Verbindungsabschnitt Bodenkontakt hätte, würde der Wirkungshebel des Prothesenfußes deutlich verringert, wodurch der Prothesenfuß beim Auftreten deutlich steifer als beim Abheben des Prothesenfußes am Ende des Bodenkontaktes wäre. Ein solches Verhalten ist unerwünscht und wird durch die konkave Krümmung posterior zu dem Auftrittsabschnitt verhindert.

Eine Weiterbildung der Erfindung sieht vor, dass der Anschlussabschnitt zumindest in dem Bereich des Aufnahmeschlitzes einen bezogen auf einen Abrollbereich im Auftrittsabschnitt konstanten Krümmungsradius aufweist, so dass unabhängig von der Position des Anschlussadapters eine konstante Fußhöhe beibehalten bleiben kann. Der Abrollbereich ist dabei derjenige Bereich, der bei der Verstellung des Anschlussadapters von maximal anterior bis maximal posterior überstrichen wird, wenn der Prothesenfuß nicht oder nur statisch belastet wird, z.B. wenn der Prothesennutzer entspannt steht. Der konstante Krümmungsradius kann dadurch erreicht werden, dass die Radien des Anschlussabschnittes und des Auftrittsabschnittes gleich sind oder aber näherungsweise der Abrollbereich so schmal ist, dass keine signifikante Veränderung der Prothesenhöhe vorliegt. Dadurch wird gewährleistet, dass über den Verstellbereich des Aufnahmeschlitzes eine konstante Prothesenfußhöhe vorliegt.

An dem Anschlussabschnitt, z.B. an dem Aufnahmeschlitz, an der Blattfeder, an der Anschlussadapterlagerung und/oder dem Anschlussadapter kann oder können eine Skalierung zur Ausrichtung der anterior-posterior-Verstellung des Anschlussadapters angeordnet sein, beispielsweise durch Strichmarkierungen und eine korrespondierende Markierung an dem Anschlussadapter, so dass durch die Skalierung eine Relation zu dem erreichten Härtegrad in quantifizierbarer Weise hergestellt wird. Dadurch ist es möglich, die Härte der Feder und damit auch den Prothesenfuß als solchen nicht nur nach Gefühl zu verstellen, sondern exakt nach gemessenen Parametern. Die Skalierung oder Markierung kann einen Nonius aufweisen. Durch die Skalierung ist eine Reproduzierbarkeit der Einstellung möglich.

Die Blattfeder ist im Anschlussabschnitt bevorzugt dicker als im Auftrittsabschnitt, um eine präzise Zuordnung des Anschlussadapters zu dem Prothesenschaft und zu der Blattfeder zu erreichen. Aufgrund der erhöhten Steifigkeit, die mit einer vergrößerten Blattfederdicke aufgrund einer erhöhten Anzahl von Webelagen und/oder einer zusätzlichen Kunststoffmatrixmasse einhergeht, wird nur eine geringe Verformung in dem Anschlussabschnitt zugelassen.

Der Aufnahmeabschnitt ist vorteilhafterweise bis zu dem Übergang in den Verbindungsabschnitt steifer als in dem Verbindungsabschnitt selbst und als in dem Auftrittsabschnitt. Die steifere Ausgestaltung, die sich bis vor der hinteren Krümmung oder dem hinteren Bogen erstrecken kann, bewirkt, dass sich die Blattfeder in diesem Bereich nur gering verformt, um so eine möglichst geringe Horizontalverschiebung der Wirkungslinie der Blattfeder zu bewirken. Eine Verformung der Blattfeder in dem Bereich des Anschlussabschnittes hätte eine nahezu horizontale Verformung der Prothesenfußspitze zur Folge, was unerwünscht ist. Erst im Bereich des hinteren konvexen Bogens wird die Blattfeder verjüngt ausgeführt, weil eine Verformung hier zu einer deutlich verringerten Horizontalverschiebung der Wirkungslinie führt. Auch eine Verformung in dem Verbindungsabschnitt mit der konkaven Formgebung führt zu einer vergleichsweise vertikaleren Wirkungsrichtung der Blattfeder und ist somit erwünscht. Über die sich von dem Anschlussabschnitt zu dem Auftrittsabschnitt hin verjüngende Feder ist es möglich, die mechanischen Belastungen innerhalb der Feder im Wesentlichen konstant zu halten, da an dem vorderen Federende, also im Bereich der Fußspitze, eine geringere mechanische Belastung als im Bereich der Abstützung im Anschlussadapter auftritt. Die steife Ausgestaltung der Blattfeder im Anschlussabschnitt und die weichere Ausgestaltung ab dem Endbereich des Anschlussabschnittes ist auch unabhängig von der Verstellbahn des Anschlussadapters als eigenständige Erfindung anzusehen. Die Verjüngung oder weichere Ausgestaltung der Feder beginnt vor dem hinteren Bogen und kann bis zu Fußspitze zunehmen. Die zunehmend weichere Ausgestaltung kann kontinuierlich oder in diskreten Schritten erfolgen, beispielsweise abschnittsweise für den Verbindungsabschnitt und den Auftrittsabschnitt.

In einem Aufnahmeschlitz, an einem Aufnahmesteg oder an der Blattfeder ist der Anschlussadapter angeordnet, wobei der Anschlussadapter eine zu dem Anschlussabschnitt beziehungsweise dem jeweiligen Befestigungsbauteil korrespondiert geformte Kontaktfläche aufweist. Dies bedingt, dass der Anschlussadapter mehrteilig ausgebildet ist und bevorzugt einen oberen und unteren Teil aufweist, was zur Folge haben kann, das die Teile einerseits eine konkave und andererseits eine konvexe Formgestaltung aufweisen, um sich an die ggf. gebogene Form des Anschlussabschnittes oder der Anschlussadapterlagerung möglichst vollflächig anlegen zu können.

Der Anschlussadapter kann wenigstens aus einem ersten, bevorzugt oberen und einem zweiten, bevorzugt unteren Adapterteil bestehen, wobei beide Adapterteile korrespondierend geformte Kontaktflächen aufweisen, so dass eine großflächige Anlage der Adapterteile an der Blattfeder oder der Adapterlagerung gewährleistet ist. Erfolgt eine Befestigung an einem Steg oder in einem darin ausgebildeten Schlitz, können die Adapterteile auch rechts und links des Steges angeordnet werden.

Um eine problemlose Verschiebung über den gesamten Verstellbereich bewirken zu können, ist in dem Anschlussabschnitt die Blattfeder mit einer konstanten Materialstärke versehen, so dass eine Verkippung des Anschlussadapters aufgrund von unterschiedlichen Materialstärken, beispielsweise einer sich verjüngenden Blattfeder im hinteren Verstellbereich, ausgeschlossen wird. Es kann auch eine seitliche Klemmung an der Blattfeder direkt erfolgen.

In dem Auftrittsabschnitt kann ein Sohlenkonturelement, das zumindest ein Polster oder eine Profilierung aufweist, befestigt sein, um die gegen Abrasion empfindliche Blattfeder zu einem Kompositmaterial gegen Verschleiß zu schützen und darüber hinaus eine verbesserte Traktion für den Prothesenfußnutzer bereitzustellen. Das Sohlenkonturelement mit Polster oder Profilierung deckt dabei vorteilhafterweise die Prothesenfußspitze ab, um gerade in den Stirnflächen der Blattfeder einen Schutz einerseits der Faserkomponenten und andererseits der Nutzer gegen scharfkantige Absplitterungen oder dergleichen vorzusehen.

Die Prothesenfußspitze überragt den Anschlussabschnitt anterior, so dass der Aufsetzpunkt oder Auftrittspunkt bzw. die Auftrittslinie des Auftrittsabschnittes vorteilhafterweise zwischen 3 cm und 5 cm vor der Lotlinie des Anschlussadapters liegt.

Die Länge des Verstellbereiches, z.B. des Aufnahmeschlitzes oder des Verschiebeweges an dem Anschlussabschnitt, erlaubt vorteilhafterweise eine Rotation zwischen 5° und 10°, was sich als ausreichend erwiesen hat, um eine hinreichende Variabilität innerhalb des Prothesenfußes bereitzustellen, ohne dass die Federlänge zu groß wird und die Materialstärke zu groß bemessen werden muss, um bei einer noch größeren Vergrößerung der Hebellänge die auftretenden Kräfte aufnehmen zu können.

Der Prothesenfuß kann mit Blattfedern in unterschiedlichen Härtegraden und Dimensionen realisiert werden, so dass er für unterschiedlich schwere Patienten und als Kinderprothesenfuß geeignet ist. Die Prothesenfußhöhe kann in Abhängigkeit von der Körpergröße und der Amputationshöhe bzw. Stumpflänge gewählt werden.
Der Verlauf der Bahn, entlang der der Anschlussadapter verstellt werden kann, ist bevorzugt so ausgebildet, dass bei einer Verlagerung der Blattfeder relativ zu dem Anschlussadapter in anteriorer-posteriorer Richtung die Prothesenfußhöhe im Wesentlichen konstant bleibt. Aufgrund der bevorzugt konvex Formung der Bahn erfolgt bei einer Verlagerung des anterioren proximalen Endes der Blattfeder entlang des Verstellweges in posteriore Richtung eine Rotation der Blattfeder, so dass der Auftrittspunkt bzw. die Auftrittslinie des blattförmigen, vorteilhafterweise ebenfalls konvex, diesmal in Bodenrichtung gewölbt, geformten Auftrittsabschnitts gleichermaßen in anteriore Richtung wandert, so dass einerseits eine konstante Prothesenfußhöhe oder zumindest eine nahezu konstante Prothesenfußhöhe bei konstanter Position des Auftrittsbereiches bereitgestellt wird. Unter einer im Wesentlichen konstanten Prothesenfußhöhe wird eine maximale Differenz von weniger als 5% der Ausgangsfußhöhe angesehen.

Der Abstand des Auftrittsabschnittes bleibt während der Verstellung des Anschlussadapters vorteilhafterweise im Wesentlichen konstant zu der Lotlinie des Anschlussadapters, insbesondere der Auftrittspunkt oder Auftrittsbereich oder die Auftrittslinie, um bei veränderbarer Federsteifigkeit einen unveränderten oder nahezu unveränderten Prothesenaufbau zu haben.

Nachfolgend werden Ausführungsbeispiele der Erfindung anhand der beigefügten Figuren näher erläutert. Es zeigen:
- Figur 1 -: eine Blattfeder eines Prothesenfußes ohne Adapter;
- Figur 2 -: einen Prothesenfuß mit Adapter;
- Figur 3 -: eine seitliche Explosionsdarstellung eines Prothesenfußes;
- Figur 3a -: eine Darstellung von Verformungsrichtungen;
- Figur 4 -: eine Detailansicht eines Adapters in Seitenansicht;
- Figur 5 -: eine Detailansicht eines montierten Adapters in Seitenansicht,
- Figur 6 -: eine Seitenansicht zweier Adapterteile;
- Figur 7 -: eine perspektivische Darstellung eines Anschlussadapters;
- Figur 8 -: eine Darstellung unterschiedlicher Einstellpositionen der Blattfeder zu dem Anschlussadapter;
- Figur 9 -: einen Prothesenfuß mit Sohlenkonturelement;
- Figur 10 -: eine Blattfeder in Einzeldarstellung;
- Figur 11 -: eine perspektivische Darstellung einer Variante mit Anschlussadapterlagerung; sowie
- Figur 12 -: eine Seitenansicht der Figur 11.

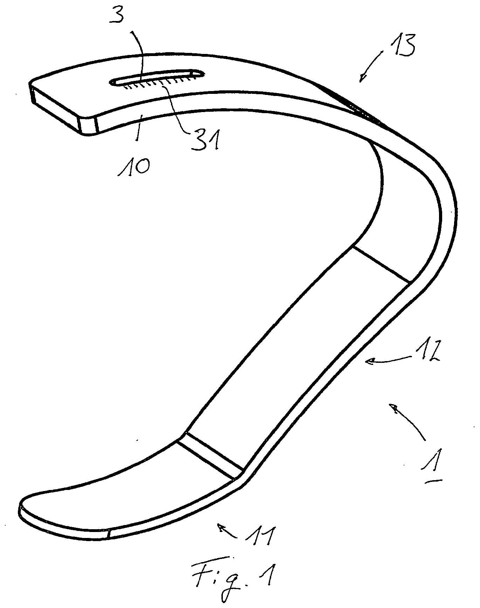

In der Figur 1 ist in einer perspektivischen Schrägansicht der Prothesenfuß 1 gezeigt, der aus einer einteiligen Blattfeder 10 besteht und einen bodenseitigen Auftrittsabschnitt 11 aufweist, der an dem distalen Ende der Blattfeder 10 angeordnet ist. Der Auftrittsabschnitt 11 ist zum Boden hin konvex gewölbt geformt, um ein Abrollen während des Laufens zu ermöglichen. An den Auftrittsabschnitt 11 schließt sich ein konkaver, vom Boden weg gewölbter Verbindungsabschnitt 12 an, der sich schräg nach hinten und oben erstreckt, also in proximal-posteriorer Richtung. Durch die konkave Ausgestaltung des Verbindungsabschnittes 12 wird dieser von einem Bodenkontakt bei einem angewinkelten Auftreten, beispielsweise bei einer deutlichen Vorverlagerung des Prothesenfußes 1 abgehalten, so dass bis zu einem Auftrittswinkel von 25° der ausschließliche Kontakt im Auftrittsabschnitt 11 sichergestellt wird. An das proximale Ende des Verbindungsabschnittes 12 schließt sich ein Anschlussabschnitt 13 an, der wiederum konvex geformt ist, in diesem Fall von dem Boden weg gekrümmt, wobei die Krümmungsrichtung des Anschlussabschnittes 13 gleich bleibt, allerdings kann sich der Krümmungsradius über die Länge des Anschlussabschnittes 13 verändern. Der Krümmungsradius verringert sich zum hinteren Ende der Blattfeder 10 hin, so dass sich ein Bogen ausbildet, durch den die Blattfeder 10 im weiteren Verlauf wieder nach vorn, in anteriore Richtung verläuft.

Im Endbereich des Anschlussabschnittes 13 ist ein in anteriorer-posteriorer Richtung verlaufender Aufnahmeschlitz 3 eingearbeitet oder ausgebildet, der zur Aufnahme des nicht dargestellten Anschlussadapters dient. Im Bereich des Aufnahmeschlitzes 3 ist eine Skalierung 31 in Gestalt von Schlitzen oder Markierungen aufgebracht, um den Anschlussadapter präzise und wiederholbar auszurichten. Der Aufnahmeschlitz 3 ist im Wesentlichen geradlinig ausgebildet und mittig in dem vorderen Ende des Anschlussabschnittes 13 als Durchgangsschlitz ausgebildet.

Die Blattfeder 10 ist einstückig aus einem Faserverbundwerkstoff ausgebildet, insbesondere als eine Feder, die mit Karbonfasern verstärkt ist. Faserverbundwerkstoffe werden in der Regel aus in Harz getränkten Faserfolienschicht, sogenannte Prepregs, hergestellt, die orientiert übereinandergelegt und unter Wärmeeinfluss miteinander verbunden werden.

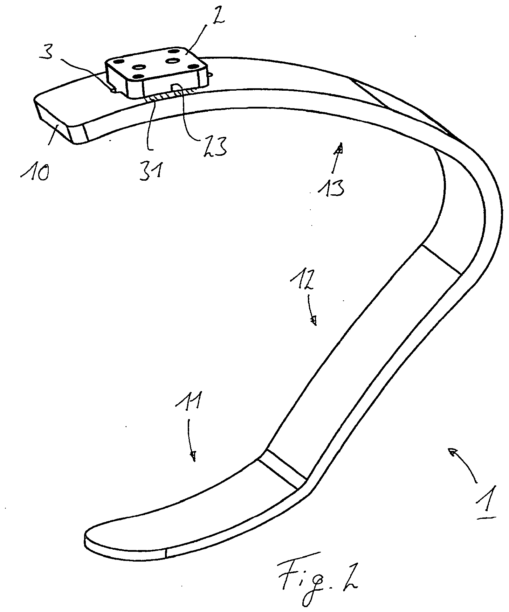

In der Figur 2 ist der Prothesenfuß 1 mit dem Anschlussadapter 2 montiert dargestellt. An dem Anschlussadapter 2 ist eine Markierung 23 angeordnet, die zu der Skalierung 31 oder den Markierungen auf dem Anschlussabschnitt 13 ausgerichtet werden kann. In der Figur 2 ist zu erkennen, dass der Auftrittsabschnitt 11 eine konstante Krümmung und eine konvexe, nach außen geölbte Form aufweist und dass der Anschlussabschnitt 13 sich dachförmig von dem proximalen Ende des Verbindungsabschnittes 12 nach vorn erstreckt. Dabei ist im posterioren Bereich des Anschlussabschnittes 13 eine stärkere Krümmung vorgesehen, damit der Anschlussabschnitt 13 sich von dem schräg nach hinten erstreckenden Verbindungsabschnitt 12 bis über den Auftrittsabschnitt 11 hinweg erstrecken kann. Im Bereich des Aufnahmeschlitzes 3 ist die Krümmung oder Wölbung der Blattfeder 10 ebenso konstant wie deren Materialstärke. Die Materialstärke der Blattfeder 10 verringert sich von dem Anschlussabschnitt 13 zum Verbindungsabschnitt 12 und wiederum zum Auftrittsabschnitt 11, wo die Blattfeder 10 am dünnsten ist.

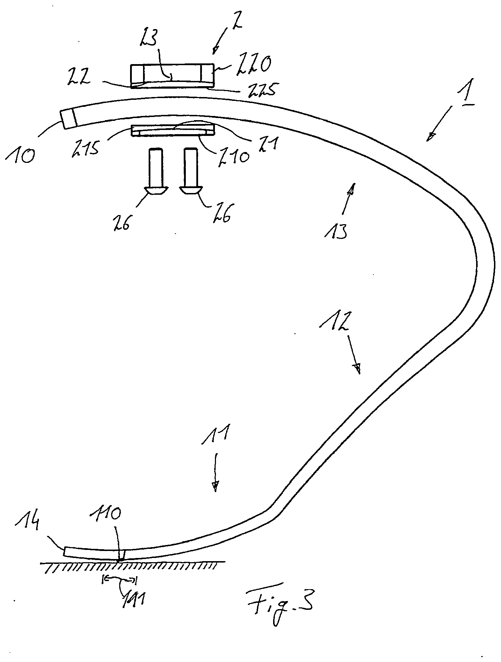

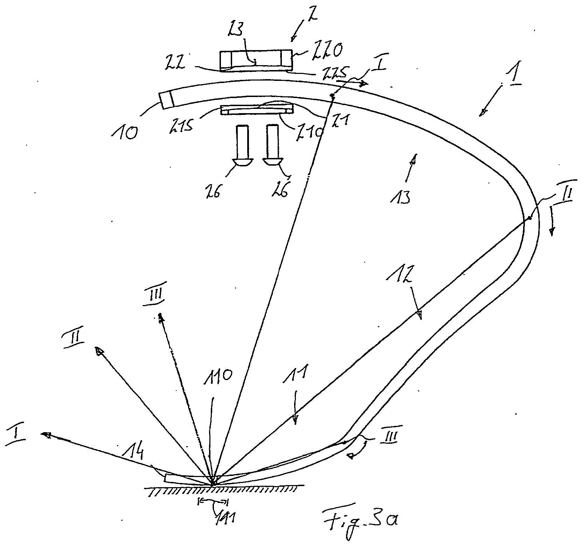

In der Figur 3 ist in einer Explosionsdarstellung der Prothesenfuß 1 in einer Seitenansicht gezeigt. Der Anschlussadapter 2 ist aus einem Oberteil 220 und einem Unterteil 210 ausgebildet, die über zwei Schrauben 26 miteinander verbindbar sind. Das Oberteil 220 ist mit einer Markierung 23 zum Ausrichten auf der Blattfeder 10 versehen. Die Kontaktoberfläche 22 des Oberteils 220 zu der Oberseite des Anschlussabschnittes 13 weist eine zu der Wölbung des Anschlussabschnittes 13 korrespondierende Krümmung auf, die entsprechend zu der konvexen Form der Blattfeder im Anschlussabschnitt 13 konkav geformt ist. Dementsprechend weist die Kontaktfläche 21 des Unterteils 210 eine konvexe Formung korrespondierend zu der Form der Unterseite des Anschlussabschnittes 13 auf. Beide Teile 220, 210 des Anschlussadapters 2 weisen Führungsstege 215, 225 auf, die im Wesentlichen der Breite des Aufnahmeschlitzes 3 entsprechen und eine Verdrehung des Anschlussadapters 2 innerhalb des Aufnahmeschlitzes 3 verhindern.

Der Anschlussadapter 2 liegt in der stehenden Ausrichtung des Prothesenfußes 1 über dem Auftrittsabschnitt 11, wobei sich der Anschlussadapter 2 innerhalb der lotrechten Projektion des Auftrittsabschnittes 11 befindet. Die Fußspitze 14 am anterioren und distalen Ende der Blattfeder 10 befindet sich vor dem Anschlussadapter 2, ebenso wie der Abrollbereich 111, der mit dem Fußboden in Kontakt tritt und innerhalb dessen der Aufsetzpunkt 110 bzw. die Aufsetzlinie bei einer gewölbten Blattfeder 10 befindlich ist.

Anhand der Figur 3a wird die Verformungsrichtung der Blattfeder 10 für Verformungen in unterschiedlichen Abschnitten verdeutlicht. Erfolgt eine Verformung in dem Anschlussbereich um den Punkt I, führt dies zu einer Verformung in einer Richtung senkrecht oder normal zu der Verbindungslinie zu dem Aufsetzpunkt 110, wie sie durch den Pfeil I dargestellt ist. Eine Verformung um den Punkt I im Anschlussabschnitt 13 wirkt sich negativ auf die Verformung der Fußspitze 14 aus, da die Verformung nahezu horizontal orientiert ist. Ziel ist es daher, die Blattfeder in diesem Bereich möglichst steif auszubilden, z.B. durch eine zusätzliche Materialstärke, Federbreite oder entsprechende Materialzusammensetzung oder Faserorientierung. Erst im Bereich des hinteren Bogens ist die Feder weicher oder dünner ausgestaltet, da eine Verformung im Punkt II deutlich weniger horizontal orientiert ist, wie an dem Pfeil II zu erkennen ist. Dadurch wird eine geringere Horizontalverschiebung der Fußspitze 14 bewirkt. Auch eine Verformung im Verbindungsabschnitt 12 zwischen dem hinteren Bogen und dem Auftrittsabschnitt 11 kann sich verformen, wie anhand der Verformung im Übergang zu dem Auftrittsabschnitt 11 in Punkt III gezeigt ist. Eine Verformung dort führt zu einer überwiegend vertikalen Verformung der Fußspitze 14, rückwärtig wirkende Kraftkomponenten sind sehr gering vorhanden. Daher ist der Bereich der Blattfeder 10 von der Adapterbefestigung bis zu dem Einlauf in den hinteren Bogen bei eine vorteilhafterweise konstanten der nahezu konstanten Dicke steif ausgelegt, während die sich distal daran anschließenden Blattfederbereiche weiche, insbesondere dünner oder schmaler ausgebildet sind.

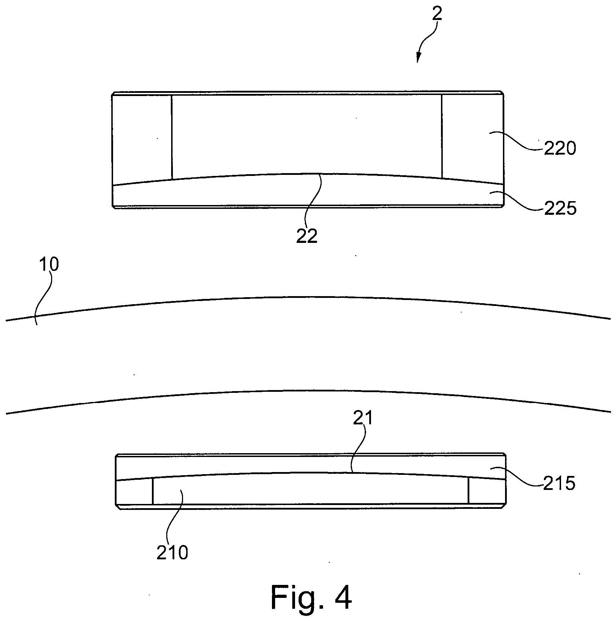

Figur 4 zeigt in einer vergrößerten Detaildarstellung den Anschlussadapter 2 mit dem Oberteil 220, dem Unterteil 210 und der dazwischen angeordneten Blattfeder 10 im Anschlussabschnitt 13. Die Blattfeder 10 ist nach oben hin konvex gekrümmt, die Kontaktflächen 22, 21 des Oberteils 220 und des Unterteils 210 sind korrespondierend zu der Oberfläche des Anschlussabschnittes 13 konkav bzw. konvex ausgebildet, der Führungssteg 215, 225 ist mittig angeordnet und entspricht in seiner Breite der Breite des nicht dargestellten Aufnahmeschlitzes.

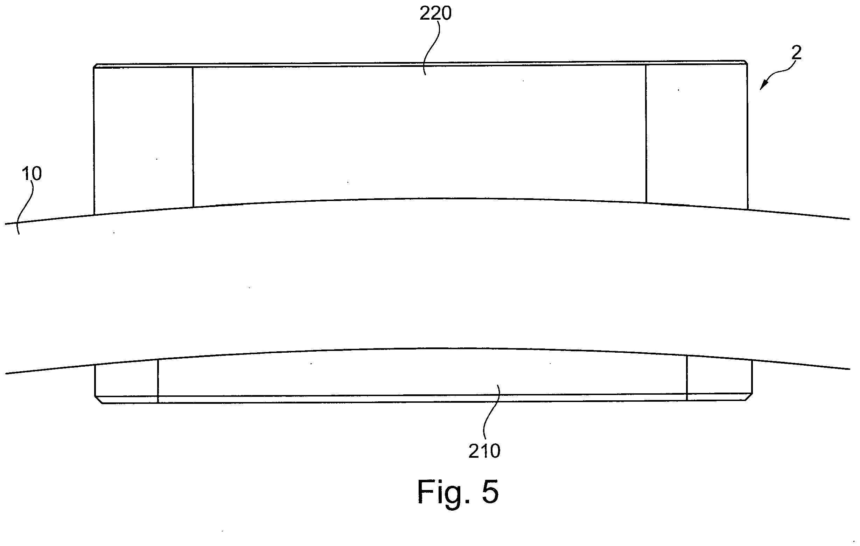

Figur 5 zeigt den Anschlussadapter 2 im montierten Zustand in Seitenansicht, die Kontaktflächen liegen bündig an der jeweiligen Oberfläche der Blattfeder 10 an.

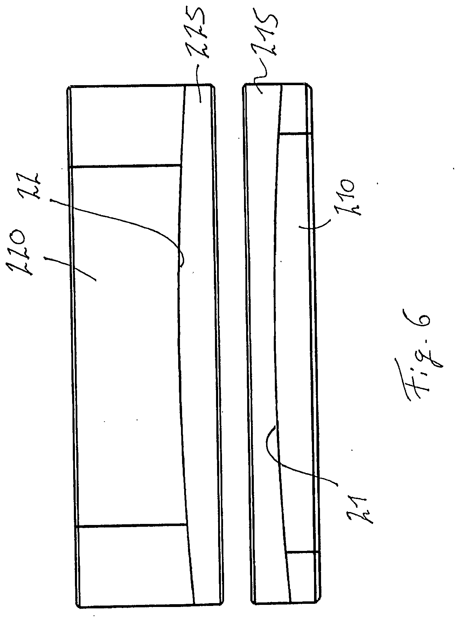

Figur 6 zeigt den Anschlussadapter 2 in Einzeldarstellung im nicht montierten Zustand, die beiden Komponenten sind in der Seitenansicht im Wesentlichen rechteckig ausgebildet, die Oberflächen der Führungsstege 215, 225 liegen parallel zueinander.

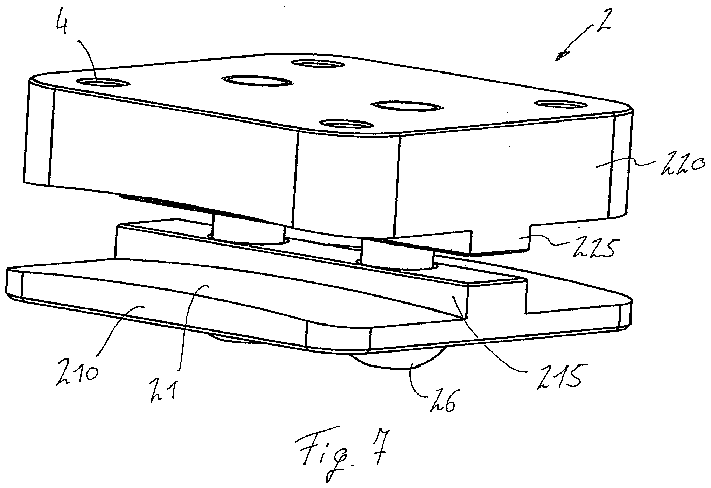

Figur 7 zeigt den Anschlussadapter 2 im zusammengesetzten Zustand ohne Blattfeder, die konvex gekrümmte Kontaktfläche 21 des Unterteils 210 ist ebenso zu erkennen wie der mittig angeordnete Führungssteg 215, die fluchtende Anordnung des Oberteils 220 und des daran ausgebildeten Führungssteges 225 sowie die beiden Befestigungsschrauben 26, die durch die Führungsstege 215, 225 hindurchgehen und in einem Gewinde in dem Oberteil 220 eingreifen. An dem Oberteil 220 sind Gewinde 4 zum Befestigen von weiteren Anschlussmitteln, beispielsweise Justierkernen, Pyramidenanschlüssen oder Rohren, vorgesehen.

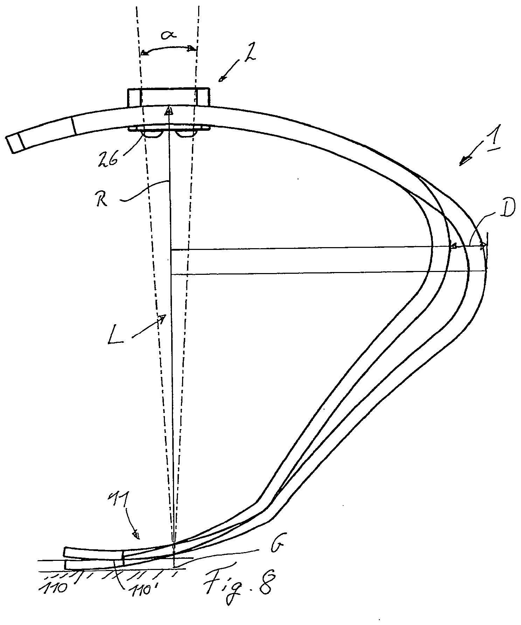

In der Figur 8 ist der Prothesenfuß 1 in zwei unterschiedlichen Ausrichtungen dargestellt. Ausgehend von einer Lotlinie L des Anschlussadapters 2 ist die Blattfeder 10 des Prothesenfußes 1 um den Winkel α verschwenkbar um den Fußpunkt der Lotlinie L, wobei der Winkel α die Gesamtlänge des Aufnahmeschlitzes 3 darstellt. Die Gesamtverstelllänge kann eine Rotation zwischen 5° und 10° ermöglichen, so dass, ausgehend von einer mittigen Anordnung, innerhalb des Aufnahmeschlitzes 3 ein Verkippen in anteriorer Richtung und posteriorer Richtung bis zu 5° in jeder Richtung möglich ist. Die beiden Darstellungen zeigen die Blattfeder 10 einmal in der weitesten nach vorn verkippten Stellung und in der weitesten nach hinten verkippten Stellung. Daraus ist zu erkennen, dass die Hebellänge, also der Abstand von der Kraftlotlinie L zum hinteren oder posterioren Ende des Anschlussabschnittes 13 unterschiedlich groß ist. Wird die Blattfeder nach hinten verkippt, vergrößert sich der Hebel, wird sie nach vorne verkippt, verringert sich der Hebel. Die Differenz D zwischen der vorderen und hinteren Stellung ist die Differenz der Hebellänge, so dass über die effektiv wirksamen Hebel das Verhalten der Blattfeder 10 eingestellt werden kann.

Der Radius R der Krümmung des Anschlussabschnittes 13 ist zumindest über den Verstellweg des Anschlussadapters 2 konstant. Im dargestellten Ausführungsbeispiel wird als Bezugsgröße die Lotlinie L und der Fußpunkt der Lotlinie L auf dem Boden herangezogen. Der Aufsetzpunkt 110 bzw. 110' liegt in Laufrichtung vor dem Fußpunkt der Lotlinie L und vor der Projektion des Anschlussadapters 2 auf den Boden. Es ist zu erkennen, dass sich eine geringe Höhendifferenz zwischen den Aufsetzpunkten 110, 110' ergibt, diese Differenz ist in der Praxis in der Regel vernachlässigbar und kann dadurch verringert oder ganz vermieden werden, wenn die Radien R der Krümmungen des Anschlussabschnittes 13 und des Auftrittsabschnittes 11 übereinstimmen oder der Aufsetzpunkt 110 mit dem Fußpunkt der Lotlinie L übereinstimmt. Weiterhin ist zu erkennen, dass die Aufsetzpunkte 110, 110' in einem nahezu konstanten Abstand vor der Lotlinie L verbleiben. Bei einer geradlinigen Ausgestaltung der Verstellbahn des Anschlussadapters 2 würde der Aufsetzpunkt jeweils um den Verstellweg nach vorn oder hinten wandern.

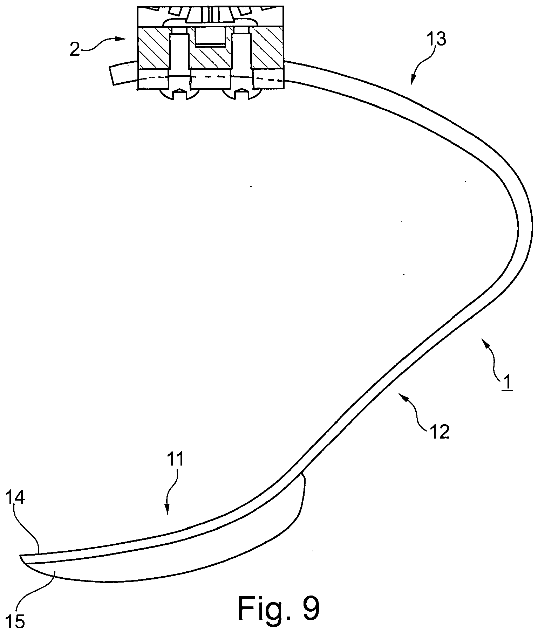

Figur 9 zeigt einen vollständigen Prothesenfuß 1 mit der Blattfeder 10, dem montierten Anschlussadapter 2 sowie einem Sohlenkonturelement mit Polster 15, das an der Unterseite des Auftrittsabschnittes 11 angeordnet ist und die Fußspitze 14 der Blattfeder 10 in vorderer Richtung überragt und abdeckt. Über das Sohlenprofilelement oder Polster 15, in dem auch eine Profiliierung eingearbeitet sein kann, kann die Position des Aufsetzpunktes 110 für jede Position des Verstellung der Blattfeder bestimmt werden. Wenn das Polster 15 auswechselbar an der Blattfeder 10 festgelegt ist, können individuelle Anpassungen einfach über Veränderungen an dem Polster ohne Veränderungen an der Blattfeder erfolgen. Bei einer Verschiebung der Blattfeder entlang der konvexen Bahn wird keine Höhenveränderung des Prothesenfußes 1 und keine Verlagerung des Aufsetzpunktes 110 bewirkt, so dass trotz Veränderung der elastischen Eigenschaften der Blattfeder 10 der Prothesenaufbau nicht verändert wird.

Durch die Formgebung der Blattfeder 10 ist es möglich, dass keine rückwärtig gerichtete Kraftkomponente beim Auftreten, Einfedern und Ausfedern auftritt. Durch die Veränderung des Hebelarms aufgrund der verschieblichen Lagerung in einem konvexen Bereich des Anschlussabschnittes ist es möglich, das Einfederverhalten und das Ausfederverhalten der Blattfeder 10 sowie die Energiespeicherfähigkeit zu verändern, wie es von dem Prothesenfußnutzer gewünscht wird. Der untere Drehpunkt kann dabei im Wesentlichen dem Auftrittspunkt bzw. der Auftrittslinie der Blattfeder im Auftrittsabschnitt entsprechen, so dass bei einem entsprechenden Radius der Krümmung im Anschlussabschnitt eine konstante Prothesenfußhöhe bereitgestellt wird.

Das Polster 15 kann an dem Auftrittsbereich 11 angeschraubt sein, um es bei Verschleiß leicht austauschen zu können. In der Seitenansicht gemäß Figur 9 ist zu erkennen, dass die Federdicke von dem Anschlussabschnitt 13, in dem die Materialstärke im Wesentlichen konstant bleibt, genauer von dem Beginn des hinteren Bogens bis zur Fußspitze 14 abnimmt. Der Krafteinleitungspunkt und die Wirkungslinie der Feder bleibt durch die erfindungsgemäße Ausgestaltung im Wesentlichen gleich, unabhängig von der Länge des wirksamen Hebels, da zusammen mit einer Verschiebung des Anschlussadapters 2 eine Rotation der Blattfeder im Wesentlichen um den Auftrittspunkt bzw. die Auftrittslinie erfolgt, allerdings kann die Energiespeicherkapazität der Blattfeder verändert werden. Durch eine Verstellung der Blattfeder 10 kann der Anstellwinkel des Prothesenfußes verändert werden, wodurch eine Variation erfolgen kann, welche Verformung durch den Kraftvektor entsteht, eine eher in Richtung des Körperschwerpunktes oder in horizontale Richtung gerichtete Verformung.

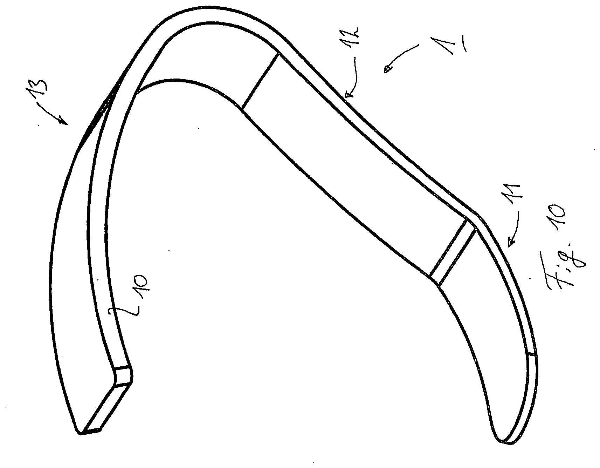

Figur 10 zeigt in einer perspektivischen Darstellung die Blattfeder 10 ohne Anschlussadapter. Der Anschlussabschnitt 13 ist mit einer konvexen, nach außen gerichteten Wölbung versehen, die korrespondierend zu der konvexen, nach unten und außen gerichteten Wölbung des Auftrittabschnitts 11 ausgebildet ist, so dass ein beispielsweise klemmend auf dem Anschlussabschnitt 13 gehaltener Anschlussadapter bei der Verstellung der Blattfeder 10 in anteriorer-posteriorer Richtung in der Höhe unverändert bleibt, da das Abrollen an der Unterseite des Auftrittsabschnitts 10 durch die Krümmung des Anschlussabschnitts 13 ausgeglichen wird. Die jeweils beteiligten Bereiche des Anschlussabschnitts 13 und des Auftrittsabschnitts 11 liegen dann auf einer Kreisbahn oder einer angenäherten Kreisbahn, so dass sich die Prothesenhöhe bzw. Prothesenfußhöhe und der Auftrittsbereich nicht verändern. Verschwenkt die Blattfeder nur um eine Achse und rollt nicht auf einer Oberfläche ab, bildet die Achse den Drehpunkt und Ursprung für den Krümmungsradius.

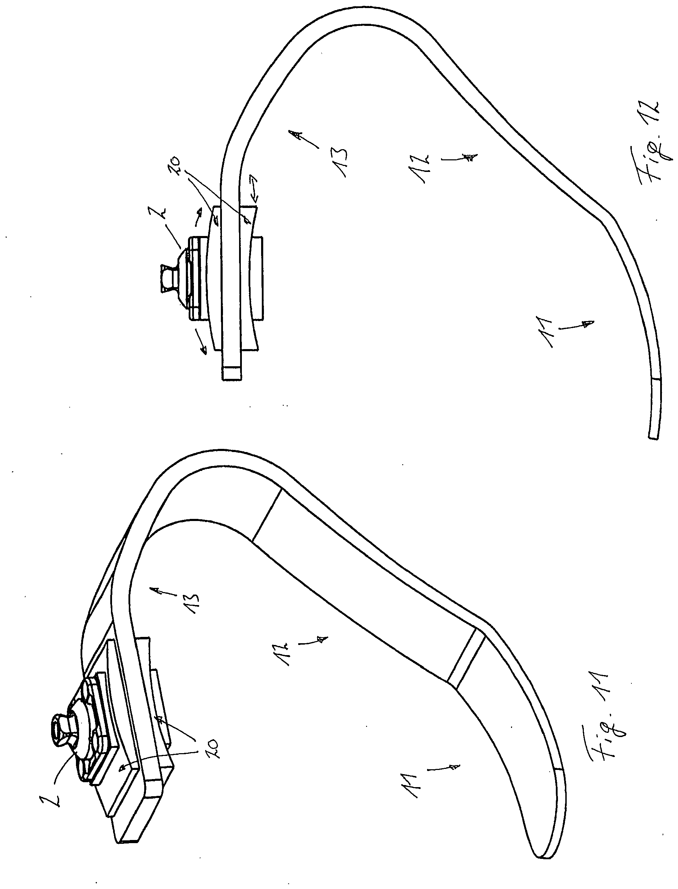

Figur 11 zeigt eine Variante der Erfindung in einer perspektivischen Schrägdarstellung. Der Anschlussabschnitt 13 ist im dargestellten Ausführungsbeispiel nicht gekrümmt ausgebildet, vielmehr ist er im Wesentlichen waagerecht zu einer nicht dargestellten Bodenfläche orientiert. An dem Anschlussabschnitt 13 ist eine Anschlussadapterlagerung 20 angeordnet, die zweiteilig ausgebildet ist, analog zu der Ausgestaltung gemäß der Figuren 1 bis 9. Die jeweilige Kontaktfläche der beiden Teile der Anschlussadapterlagerung 20 sind eben ausgebildet, so dass sie vollflächig auf der ebenen Fläche des Anschlussabschnitts 13 aufliegen. Sollte der Anschlussabschnitt 13 eine Krümmung aufweisen, ist die jeweilige Kontaktfläche des jeweiligen Teils der Anschlussadapterlagerung 20 korrespondierend gekrümmt. Auf der Anschlussadapterlagerung 20 ist der Anschlussadapter 2 beweglich gelagert. Die Oberfläche der Oberseite des oberen Anschlussadapterlagerungsteils ist konvex gekrümmt, so dass bei einer Verlagerung des Anschlussadapters 2 in anteriorer-posteriorer Richtung der Anschlussadapter 2 auf einer nach oben gebogenen, nach außen gewölbten Bahn entlang gleitet. Die Anschlussadapterlagerung 20 selbst kann ebenfalls verstellbar oder verschieblich an der Blattfeder 10 im Bereich des Anschlussabschnitts 13 gelagert sein, beispielsweise in einem Schlitz, um eine Längsverstellung zu bewirken. Eine Klemmung an der Blattfeder 10 kann über Befestigungsschrauben des Anschlussadapters 10 oder über separate Klemmeinrichtungen, beispielsweise Schrauben vorgenommen werden. An der Anschlussadapterlagerung 20 können analog zu dem Anschlussadapter 2 gemäß Figur 2 ebenfalls eine Skalierung vorgesehen sein, um eine Einstellung entweder der Anschlussadapterlagerung 20 an der Blattfeder 10 oder die Orientierung des Anschlussadapters 2 an der Anschlussadapterlagerung 20 zu erleichtern.

Figur 12 zeigt die Variante gemäß Figur 11 in einer Seitenansicht. Die jeweils konvexe Krümmung einmal des Auftrittsabschnitts 11 und einmal der Anschlussadapterlagerung 20 zur bogenförmigen Verlagerung des Anschlussadapters 2 bei einer Verstellung der Blattfeder 10 in anteriorer-posteriorer Richtung ist zu erkennen. Die Anschlussadapterlagerung 20 kann in anteriorer-posteriorer Richtung an der Blattfeder 10 verlagert werden. Alternativ zu einer Befestigung des Anschlussadapters 2 von oben ist es möglich, an der Anschlussadapterlagerung 20 einen vertikal orientierten Steg vorzusehen, an dem der Anschlussadapter 2 verschieblich gelagert und festgeklemmt werden kann. Statt einer stufenlosen Verlagerung über einen Schlitz an oder in der Anschlussadapterlagerung 20, in einem Schlitz in der Blattfeder 10 bzw. in einer Kombination davon ist es möglich, eine Verstellung in diskreten Schritten vorzunehmen oder eine Klemmung entsprechend der Blattfeder gemäß Figur 10 ohne Aufnahmeschlitz durchzuführen.

## Patentansprüche

1. Gelenkloser Prothesenfuß mit einer Blattfeder (10), die einen distalen Auftrittsabschnitt (11), einen Verbindungsabschnitt (12) und einen proximalen, sich in anterior-posterior-Richtung erstreckenden Anschlussabschnitt (13) aufweist, an dem ein Anschlussadapter (2) festlegbar ist, **dadurch gekennzeichnet, dass** der Anschlussadapter (2) entlang einer konvex gekrümmten Bahn in anterior-posterior-Richtung verstellbar gelagert ist und die Bahn zumindest bereichsweise einen Krümmungsradius (R) aufweist, der in dem Auftrittsabschnitt (11) mündet.

2. Prothesenfuß nach Anspruch 1, **dadurch gekennzeichnet, dass** der Anschlussabschnitt (13) und/oder eine Anschlussadapterlagerung (20) konvex geformt ist.

3. Prothesenfuß nach Anspruch 1 oder 2, **dadurch gekennzeichnet, dass** in dem Anschlussabschnitt (13) und/oder einer Anschlussadapterlagerung (20) ein sich in anterior-posterior Richtung erstreckender Aufnahmeschlitz (3) oder eine Aufnahmeführung für den Anschlussadapter (2) angeordnet oder ausgebildet ist.

4. Prothesenfuß nach einem der voranstehenden Ansprüche, **dadurch gekennzeichnet, dass** der Krümmungsradius (R) in einem Abrollbereich (111) in dem Auftrittsabschnitt (11) mündet.

5. Prothesenfuß nach einem der voranstehenden Ansprüche, **dadurch gekennzeichnet, dass** der Auftrittsabschnitt (11) konvex geformt ist.

6. Prothesenfuß nach einem der voranstehenden Ansprüche, **dadurch gekennzeichnet, dass** der Verbindungsabschnitt (12) konkav geformt ist.

7. Prothesenfuß nach einem der voranstehenden Ansprüche, **dadurch gekennzeichnet, dass** der Anschlussabschnitt (13) einen bezogen auf einen Abrollbereich (111) im Auftrittsabschnitt (11) konstanten Krümmungsradius (R) aufweist.

8. Prothesenfuß nach einem der voranstehenden Ansprüche, **dadurch gekennzeichnet, dass** an dem Anschlussabschnitt (13), einer Anschlussadapterlagerung (20) und/oder dem Anschlussadapter (2) eine Skalierung (31) zur Ausrichtung der anterior-posterior-Verstellung des Anschlussadapters (2) angeordnet ist.

9. Prothesenfuß nach einem der voranstehenden Ansprüche, **dadurch gekennzeichnet, dass** die Blattfeder (10) im Anschlussabschnitt (13) dicker als im Auftrittsabschnitt (11) ist.

10. Prothesenfuß nach einem der voranstehenden Ansprüche, **dadurch gekennzeichnet, dass** die Blattfeder (10) im Anschlussabschnitt (13) bis zum Übergang zu dem Verbindungsabschnitt (12) steifer als im Verbindungsabschnitt (12) und dem Auftrittsabschnitt (11) ausgebildet ist.

11. Prothesenfuß nach einem der voranstehenden Ansprüche, **dadurch gekennzeichnet, dass** an oder in dem Aufnahmeabschnitt (13) der Anschlussadapter (2) angeordnet ist und der Anschlussadapter (2) eine zu dem Anschlussabschnitt (13) korrespondierend geformte Kontaktfläche (21, 22) aufweist.

12. Prothesenfuß nach einem der voranstehenden Ansprüche, **dadurch gekennzeichnet, dass** der Anschlussadapter (2) oder eine Anschlussadapterlagerung (20) wenigstens aus einem oberen und einem unteren Adapterteil (210, 220) besteht und beide Adapterteile (210, 220) korrespondierend geformte Kontaktflächen (21, 22) aufweisen.

13. Prothesenfuß nach einem der voranstehenden Ansprüche, **dadurch gekennzeichnet, dass** in dem Anschlussabschnitt (13) die Blattfeder (10) eine konstante Materialstärke aufweist.

14. Prothesenfuß nach einem der voranstehenden Ansprüche, **dadurch gekennzeichnet, dass** im Auftrittsabschnitt (11) ein Polster (15) oder eine Profilierung befestigt ist.

15. Prothesenfuß nach Anspruch 14, **dadurch gekennzeichnet, dass** das Polster (15) oder die Profilierung die Prothesenfußspitze (14) abdeckt.

16. Prothesenfuß nach einem der voranstehenden Ansprüche, **dadurch gekennzeichnet, dass** die Prothesenfußspitze (14) den Anschlussabschnitt (13) anterior überragt.

17. Prothesenfuß nach einem der voranstehenden Ansprüche, **dadurch gekennzeichnet, dass** der Aufsetzpunkt (110, 110') des Prothesenfußes vor einer Lotlinie (L) des Anschlussadapters (2) liegt.

18. Prothesenfuß nach einem der voranstehenden Ansprüche, **dadurch gekennzeichnet, dass** der Verstellbereich des Anschlussadapters (2) eine Rotation zwischen 5° und 10° ermöglicht.

19. Prothesenfuß nach einem der voranstehenden Ansprüche, **dadurch gekennzeichnet, dass** bei einer Verlagerung der Blattfeder (10) relativ zu dem Anschlussadapter (2) in anteriorer- posteriorer Richtung die Prothesenfußhöhe im Wesentlichen konstant bleibt.

20. Prothesenfuß nach einem der voranstehenden Ansprüche, **dadurch gekennzeichnet, dass** der Abstand des Auftrittsabschnittes (11) während der Verstellung des Anschlussadapters im Wesentlichen konstant zu der Lotlinie (L) des Anschlussadapters (2) bleibt.

## Claims

1. Jointless prosthetic foot with a leaf spring (10) which has a distal foot-strike section (11), a connecting section (12), and a proximal attachment section (13) which extends in an anterior-posterior direction and on which an attachment adapter (2) can be secured, **characterized in that** the attachment adapter (2) is mounted so as to be adjustable along a convexly curved path in the anterior-posterior direction and the path has, at least regionally, a radius of curvature (R) which opens out in the foot-strike section (11).

2. The prosthetic foot as claimed in claim 1, **characterized in that** the attachment section (13) and/or an attachment adapter bearing (20) has a convex shape.

3. The prosthetic foot as claimed in claim 1 or 2, **characterized in that** a receiving slit (3), extending in the anterior-posterior direction, or a receiving guide for the attachment adapter (2) is arranged or formed in the attachment section (13) and/or in an attachment adapter bearing (20).

4. The prosthetic foot as claimed in one of the preceding claims, **characterized in that** the curvature radius (R) opens out in a roll-off area (111).

5. The prosthetic foot as claimed in one of the preceding claims, **characterized in that** the foot-strike section (11) has a convex shape.

6. The prosthetic foot as claimed in one of the preceding claims, **characterized in that** the connecting section (12) has a concave shape.

7. The prosthetic foot as claimed in one of the preceding claims, **characterized in that** the attachment section (13) has a radius of curvature (R) which is constant with respect to a roll-off area (111) in the foot-strike section (11).

8. The prosthetic foot as claimed in one of the preceding claims, **characterized in that** a scale (31) for the orientation of the anterior-posterior adjustment of the attachment adapter (2) is arranged on the attachment section, on an attachment adapter bearing (20) and/or on the attachment adapter (2).

9. The prosthetic foot as claimed in one of the preceding claims, **characterized in that** the leaf spring (10) is thicker in the attachment section (13) than in the foot-strike section (11).

10. The prosthetic foot as claimed in one of the preceding claims, **characterized in that** the leaf spring (10) in the attachment section (13), as far as the transition to the connecting section (12), is stiffer than in the connecting section (12) and in the foot-strike section (11).

11. The prosthetic foot as claimed in one of the preceding claims, **characterized in that** the attachment adapter (2) is arranged on or in the receiving section (13), and the attachment adapter (2) has a contact surface (21, 22) with a shape corresponding to the attachment section (13).

12. The prosthetic foot as claimed in one of the preceding claims, **characterized in that** the attachment adapter (2) or an attachment adapter bearing (20) is composed at least of an upper and a lower adapter part (210, 220), and both adapter parts (210, 220) have correspondingly shaped contact surfaces (21, 22).

13. The prosthetic foot as claimed in one of the preceding claims, **characterized in that** the leaf spring (10) has a constant material thickness in the attachment section (13).

14. The prosthetic foot as claimed in one of the preceding claims, **characterized in that** a padding (15) or a profiling is secured in the foot-strike section (11).

15. The prosthetic foot as claimed in claim 14, **characterized in that** the padding (15) or the profiling covers the prosthetic foot tip (14).

16. The prosthetic foot as claimed in one of the preceding claims, **characterized in that** the prosthetic foot tip (14) protrudes anteriorly beyond the attachment section (13).

17. The prosthetic foot as claimed in one of the preceding claims, **characterized in that** the set-down point (110, 110') of the prosthetic foot lies in front of a perpendicular line (L) of the attachment adapter (2).

18. The prosthetic foot as claimed in one of the preceding claims, **characterized in that** the adjustment range of the attachment adapter (2) permits a rotation of between 5° and 10°.

19. The prosthetic foot as claimed in one of the preceding claims, **characterized in that** the prosthetic foot height remains substantially constant when the leaf spring (10) shifts relative to the attachment adapter (2) in the anterior-posterior direction.

20. The prosthetic foot as claimed in one of the preceding claims, **characterized in that** the distance of the foot-strike section (11) during the adjustment of the attachment adapter remains substantially constant with respect to the perpendicular line (L) of the attachment adapter (2).

## Revendications

1. Prothèse de pied sans articulation, comportant un ressort à lame (10) qui présente une portion de pose de pied distale (11), une portion de liaison (12) et une portion de raccordement proximale (13) qui s'étend en direction antérieure - postérieure et sur laquelle peut être immobilisé un adaptateur de raccordement (2),
**caractérisée en ce que**
l'adaptateur de raccordement (2) est monté mobile en direction antérieure - postérieure le long d'une trajectoire incurvée convexe et la trajectoire présente au moins localement un rayon de courbure (R) qui débouche dans la portion de pose (11).

2. Prothèse de pied selon la revendication 1,
**caractérisée en ce que**
la portion de raccordement (13) et/ou une monture d'adaptateur de raccordement (20) est en forme convexe.

3. Prothèse de pied selon la revendication 1 ou 2,
**caractérisée en ce que**
dans la portion de raccordement (13) et/ou dans une monture d'adaptateur de raccordement (20) est disposé(e) ou ménagé(e) une fente de logement (3) ou un guidage de logement pour l'adaptateur de raccordement (2), qui s'étend en direction antérieure - postérieure.

4. Prothèse de pied selon l'une des revendications précédentes,
**caractérisée en ce que**
le rayon de courbure (R) débouche dans une zone de roulement (111) dans la portion de pose (11).

5. Prothèse de pied selon l'une des revendications précédentes,
**caractérisée en ce que**
la portion de pose (11) est de forme convexe.

6. Prothèse de pied selon l'une des revendications précédentes,
**caractérisée en ce que**
la portion de liaison (12) est de forme concave.

7. Prothèse de pied selon l'une des revendications précédentes,
**caractérisée en ce que**
la portion de raccordement (13) présente un rayon de courbure (R) constant par référence à une zone de roulement (111) dans la portion de pose (11).

8. Prothèse de pied selon l'une des revendications précédentes,
**caractérisée en ce que**
sur la portion de raccordement (13), sur une monture d'adaptateur de raccordement (20) et/ou sur l'adaptateur de raccordement (2), il est prévu une échelle (31) agencée pour orienter le déplacement antérieur - postérieur de l'adaptateur de raccordement (2).

9. Prothèse de pied selon l'une des revendications précédentes,
**caractérisée en ce que**
dans la portion de raccordement (13), le ressort à lame (10) est plus épais que dans la portion de pose (11).

10. Prothèse de pied selon l'une des revendications précédentes,
**caractérisée en ce que**
dans la portion de raccordement (13) jusqu'à la transition vers la portion de liaison (12), le ressort à lame (10) est plus rigide que dans la portion de liaison (12) et que dans la portion de pose (11).

11. Prothèse de pied selon l'une des revendications précédentes,
**caractérisée en ce que**
l'adaptateur de raccordement (2) est agencé sur ou dans la portion de logement (13), et l'adaptateur de raccordement (2) présente une surface de contact (21, 22) de forme correspondante à celle de la portion de raccordement (13).

12. Prothèse de pied selon l'une des revendications précédentes,
**caractérisée en ce que**
l'adaptateur de raccordement (2) ou une monture d'adaptateur de raccordement (20) est constitué(e) par au moins une partie d'adaptateur supérieure et par une partie d'adaptateur inférieure (210, 220), et les deux parties d'adaptateur (210, 220) présentent des surfaces de contact (21, 22) de forme correspondante.

13. Prothèse de pied selon l'une des revendications précédentes,
**caractérisée en ce que**
le ressort à lame (10) présente une épaisseur de matériau constante dans la portion de raccordement (13).

14. Prothèse de pied selon l'une des revendications précédentes,
**caractérisée en ce que**
un rembourrage (15) ou un profilage est fixé dans la portion de pose (11).

15. Prothèse de pied selon la revendication 14,
**caractérisée en ce que**
le rembourrage (15) ou le profilage recouvre la pointe (14) de la prothèse de pied.

16. Prothèse de pied selon l'une des revendications précédentes,
**caractérisée en ce que**
la pointe (14) de la prothèse de pied dépasse antérieurement au-delà de la portion de raccordement (13).

17. Prothèse de pied selon l'une des revendications précédentes,
**caractérisée en ce que**
le point d'application (110, 110') de la prothèse de pied se trouve en avant d'une ligne d'aplomb (L) de l'adaptateur de raccordement (2).

18. Prothèse de pied selon l'une des revendications précédentes,
**caractérisée en ce que**
la zone de déplacement de l'adaptateur de raccordement (2) permet une rotation entre 5° et 10°.

19. Prothèse de pied selon l'une des revendications précédentes,
**caractérisée en ce que**
lors d'un déplacement du ressort à lame (10) par rapport à l'adaptateur de raccordement (2) en direction antérieure - postérieure, la hauteur de la prothèse de pied reste sensiblement constante.

20. Prothèse de pied selon l'une des revendications précédentes,
**caractérisée en ce que**
pendant le déplacement de l'adaptateur de raccordement, la distance de la portion de pose (11) à la ligne d'aplomb (L) de l'adaptateur de raccordement (2) reste sensiblement constante.
